# EUROPEAN PATENT APPLICATION

(11) **EP 1 806 588 A1**
(43) Date of publication of application: **11.07.2007**
(21) Application number: 05805352.1
(22) Date of filing: 28.10.2005
(51) Int. Cl.: G01N 35/04, G01N 27/28

(54) **ANALYZER, CARTRIDGE, AND ANALYSIS KIT**

(30) Priority: 29.10.2004 JP 2004315638
(71) Applicant: Arkray, Inc., Kyoto-shi, Kyoto 601-8045 (JP)
(72) Inventor: Uehata, Yoshiharu c/o Arkray Inc., Kyoto-shi, Kyoto; 6018045 (JP); Hata, Hitoshi c/o Arkray Inc., Kyoto-shi, Kyoto; 6018045 (JP); Nakanishi, Hiroyuki c/o Arkray Inc., Kyoto-shi, Kyoto; 6018045 (JP)
(74) Representative: Pluckrose, Anthony William
(86) International application number: PCT/JP2005/019905
(87) International publication number: WO 2006/046701

(57) **Abstract**

The present invention relates to an analyzer (3) for analyzing a sample by taking an analytical instrument (20) out of a cartridge accommodating a plurality of analytical instruments and using the analytical instrument. The analyzer (3) includes a housing (4), an operational member (5) which is reciprocally movable relative to the housing, and a movable member (60) which moves, in accordance with the reciprocal movement of the operational member (5), reciprocally between a wait position and a take-out position for taking an analytical instrument (20) out of the cartridge. The movable member (60) is designed to engage an analytical instrument accommodated in the cartridge and take the analytical instrument (20) out of the cartridge.

## Description

### TECHNICAL FIELD

The present invention relates to a technique for analyzing a sample using an analytical instrument.

### BACKGROUND ART

A conventional method of checking the glucose level in the blood may use an analytical instrument. For instance, the bloodglucose level can be automatically measured with a portable blood glucose measuring apparatus, to which an analytical instrument is mounted by the user. Then, sample blood is applied to the analytical instrument. As another example, a cartridge accommodating a plurality of analytical instruments is used in combination with a blood glucose measuring apparatus. The analytical instruments are fed automatically from the cartridge to the blood glucose measuring apparatus, and the checking of the blood glucose level is performed automatically (See Patent Document 1, for example).

However, to mount an analytical instrument to a blood glucose measuring apparatus is a troublesome work for the user. Specifically, to use the blood glucose measuring apparatus, the user needs to take an analytical instrument out of a container or package and then mount the analytical instrument to the measuring apparatus. Particularly, in using a portable blood glucose measuring apparatus, the analytical instrument to be used is small (thin) and the portion of the measuring apparatus to which the analytical instrument is to be mounted is small. Therefore, the work to mount the analytical instrument is troublesome. Further, after the use of the mounted analytical instrument, the user needs to remove the analytical instrument from the blood glucose measuring apparatus, which is also troublesome. Moreover, in removing the analytical instrument from the blood glucose measuring apparatus, the user needs to touch the portion of the analytical instrument to which blood is applied or the nearby portion, which is not hygienic. Particularly when the analytical instrument is small, it is highly possible that the user touches the portion of the analytical instrument to which blood is applied.

On the other hand, in using a measuring apparatus combined with a cartridge, it is not necessary to touch the analytical instrument in measuring the blood glucose level. However, it is necessary to manufacture a cartridge having a complicated structure and to design a measuring apparatus to be applicable to the cartridge. Therefore, in the conventional method to combine a cartridge, the apparatus cost and the manufacturing cost are disadvantageously high.

Patent Document 1: JP-A1-01-63272

### DISCLOSURE OF THE INVENTION

An object of the present invention is to make it possible to perform sample analysis easily and hygienically by employing a structure which can be manufactured cheaply.

According to a first aspect of the present invention, there is provided an analyzer for analyzing a sample by using an analytical instrument taken out of a cartridge accommodating a plurality of analytical instruments. The analyzer comprises an operational member which is reciprocally movable relative to a housing, and a movable member which reciprocates, in accordance with the reciprocal movement of the operational member, between a wait position and a take-out position for taking an analytical instrument out of the cartridge. The movable member is configured to come into engagement with an analytical instrument accommodated in the cartridge for taking the analytical instrument out of the cartridge, with at least part of the analytical instrument received in the housing.

The operational member may move due to a load applied thereto by the cartridge when the cartridge is mounted to the analyzer while being positioned relative to the analyzer.

The movable member may include at least one arm for engagement with the analytical instrument. In this case, for instance, when the cartridge is mounted to the analyzer, the at least one arm is inserted into the cartridge to engage an analytical instrument accommodated in the cartridge, and when the cartridge is detached from the analyzer, the at least one arm pulls and takes the analytical instrument out of the cartridge and guides at least part of the analytical instrument into the housing.

The analyzer may further comprise a detector for detecting whether or not the movable member is located at an appropriate position.

The detector may include at least one switch. In this case, the movable member includes at least one detection target portion for causing the at least one switch to generate an ON signal. The detector may include a plurality of switches and detect the position of the movable member based on the combination of signals generated by the switches. Alternatively, the detector may detect the position of the movable member by utilizing a photosensor instead of a switch.

Each of the operational member and the movable member may include a plurality of cogs, and the operational member and the movable member may be connected to each other via a gear. In this case, when the operational member moves, the movable member moves in an opposite direction from the operational member.

The analyzer further comprises a stopper which restricts the movement of the movable member toward the wait position and positions the movable member at an analysis reference position set between the take-out position and the wait position when the analytical instrument taken out from the cartridge is held in engagement with the movable member.

The movable member may include a pivotable portion which pivots in a direction crossing the movement direction of the movable member to apply a force in the crossing direction to the analytical instrument taken out of the cartridge when the movable member moves reciprocally.

The analyzer further comprises a fixation element fixed to the housing. One of the pivotable portion and the fixation element includes a guide groove, whereas the other one of the pivotable portion and the fixation element includes a projection for engagement with the guide groove. In this case, when the movable member moves reciprocally relative to the housing, the projection moves within the guide groove so that the pivotable portion pivots.

The analyzer according to the present invention may further comprise an information recognizer for recognizing information as to the analytical instrument. For instance, the information recognizer includes a movable portion which is movable when the cartridge is mounted to the analyzer and a switch to be turned on or off by the movable portion.

According to a second aspect of the present invention, there is provided a cartridge for accommodating a plurality of analytical instruments to be used for sample analysis at an analyzer. The cartridge is configured so that the analytical instruments are taken out by the analyzer when the cartridge is mounted to the analyzer. The cartridge comprises a main body for accommodating the analytical instruments, a discharge port used for taking out the analytical instruments, and a selector for enabling selection between a state in which the discharge port is exposed and a state in which the discharge port is not exposed.

The selector may be rotated or slid to enable selection between the state in which the discharge port is exposed and the state in which the discharge port is not exposed. For instance, the selector may include a rotational member which is capable of performing relative rotation. The selector may include an informative portion to which information as to the analytical instrument is applied.

Preferably, the informative portion is capable of outputting information to the analyzer when the cartridge is mounted to the analyzer with the discharge port exposed. Intended information is applied to the informative portion by selecting whether or not a recess or a projection is to be formed at each of a plurality of predetermined regions of the selector. The informative portion may be provided at a portion other than the selector.

When the analyzer includes a movable member which is reciprocally movable to take an analytical instrument out of the cartridge and capable of being inserted through the discharge port, each of the analytical instruments includes an engagement portion for engagement with the movable member. For instance, the engagement portion may comprise a cutout.

The cartridge further comprises a guide for controlling the positional relationship between the cartridge and the analyzer in mounting the cartridge to the analyzer and enabling the cartridge to be mounted to an appropriate portion of the analyzer.

According to a third aspect of the present invention, there is provided an analytical kit comprising an analyzer for analyzing a sample by using an analytical instrument, and a cartridge accommodating a plurality of analytical instruments to be fed to the analyzer. The analyzer comprises an operational member which is reciprocally movable relative to a housing, and a movable member which reciprocates, in accordance with the reciprocal movement of the operational member, between a wait position and a take-out position for taking an analytical instrument out of the cartridge. The movable member is configured to come into engagement with an analytical instrument accommodated in the cartridge for taking the analytical instrument out of the cartridge, with at least part of the analytical instrument received in the housing.

The analytical instrument may include an engagement portion for engagement with the movable member. For instance, the engagement portion comprises a cutout. In this case, the movable member includes at least one arm for engagement with the cutout.

The analyzer and the cartridge include mounting means for mounting the cartridge to an appropriate position of the analyzer. For instance, the mounting means comprises a projectionprovided at one of the analyzer and the cartridge and a recess provided at the other one of the analyzer and the cartridge.

When the cartridge is mounted to the analyzer by utilizing the mounting means, the movable member may move due to a load applied to the operational member by the cartridge.

Preferably,the cartridge further comprises an informative portion to which information as to the analytical instrument is applied, and the analyzer further comprises an information recognizer for recognizing the information applied to the informative portion.

The cartridge may include a main body for accommodating the analytical instruments, a discharge port used for taking out the analytical instruments, and a selector for enabling selection between a state in which the discharge port is exposed and a state in which the discharge port is not exposed. In this case, the informative portion can be provided at the selector.

The selector may include a rotational member for enabling the selection between the state in which the discharge port is exposed and the state in which the discharge port is not exposed by rotating. Preferably, in this case, the informative portion is provided at the rotational member and outputs the information to the information recognizer when the cartridge is mounted to the analyzer with the discharge port exposed.

The information recognizer may include a plurality of movable portions which are movable when the cartridge is mounted to the analyzer, and switches to be turned on or off by the movable portions. Intended information is applied to the informative portion by selecting whether or not a recess or a projection is to be formed at each of a plurality of regions which are predetermined in the rotational member at positions corresponding to the movable portions.

Information as to the sensitivity of the analytical instrument may be applied to the informative portion. In this case, the information recognizer performs output for enabling selection of a corresponding calibration curve from a plurality of predetermined calibration curves based on the information.

According to a fourth aspect of the present invention, there is provided an analyzer for use with an analytical instrument mounted thereto, where the analyzer comprises a disposal mechanism for disposing of the analytical instrument mounted to the analyzer. The disposal mechanism comprises an operational member which is reciprocally movable relative to a housing, and a movable member for disposing of the analytical instrument by moving at least partially in accordance with the reciprocal movement of the operational member.

The movable member may reciprocate, in accordance with reciprocal movement of the operational member, between a wait position and a disposal position at which the analytical instrument is to be disposed of.

Preferably, the disposal mechanism is configured so that, when the operational member is moved in a load inputting direction from the disposal position toward the wait position, the movable member moves in a disposal direction from the wait position toward the disposal position which is opposite to the load inputting direction. In this case, the analyzer further comprises a link member for transmitting a load inputted to the operational member to the movable member. Specifically, when each of the operational member and the movable member includes a rack portion including a plurality of cogs, the link member comprises a gear meshing with the cogs. Further, the link member may include a first engagement portion engaging the operational member and a second engagement portion engaging the movable member and may be rotatable about an intermediate portion between the first and the second engagement portions.

The movable member may be a rotational cam capable of engaging the analytical instrument and the operational member. For instance, due to the movement of the operational member, the rotational cam rotates and changes, by the rotation, an engagement position with the analytical instrument to move the analytical instrument.

Preferably, the operational member is biased in the disposal direction when moved in the load inputting direction.

### BRIEF DESCRIPTION OF THE DRAWINGS

Fig. 1 is a perspective view showing an analytical kit according to a first embodiment of the present invention.
Fig. 2 includes a sectional view of a cartridge of the analytical kit shown in Fig. 1.
Fig. 3 includes a perspective view for describing the operation of the cartridge shown in Fig. 2.
Fig. 4 is an exploded perspective view of an analyzer of the analytical kit shown in Fig. 1.
Fig. 5 includes a sectional view of a principal portion of the analyzer shown in Fig. 4.
Fig. 6 includes a plan view of a principal portion of the analyzer shown in Fig. 4.
Fig. 7 is a perspective view of an operational member of the analyzer shown in Fig. 4.
Fig. 8 is a sectional view of the operational member shown in Fig. 7.
Fig. 9 is an exploded perspective view of an extracting mechanism of the analyzer shown in Fig. 4.
Fig. 10 is a plan view of a principal portion of the cartridge shown in Fig. 2.
Fig. 11 includes a sectional view of a principal portion of the cartridge for describing the operation to take out an analytical instrument from the cartridge shown in Fig. 2.
Fig. 12 is a graph showing an example of signal outputted from a switch of the analyzer shown in Fig. 4.
Fig. 13 is a perspective view showing an analytical kit according to a second embodiment of the present invention.
Fig. 14A is an overall perspective view of a cartridge of the analytical kit shown in Fig. 1, whereas Fig. 14B is a sectional view thereof.
Fig. 15 is a front view of the cartridge shown in Fig. 14.
Fig. 16 is a plan view of a principal portion of the cartridge shown in Fig. 14.
Fig. 17 is a side view of a principal portion of the cartridge shown in Fig. 14.
Fig. 18 is an exploded perspective view of the analyzer of the analytical kit shown in Fig. 13.
Fig. 19 is an exploded perspective view of an extracting mechanism of the analyzer shown in Fig. 18.
Fig. 20 includes a plan view of a principal portion of the analyzer shown in Fig. 18.
Fig. 21 is a bottom view of a principal portion of an extracting mechanism shown in Fig. 19.
Fig. 22 includes a sectional view of a principal portion of the analyzer shown in Fig. 13.
Fig. 23 includes a perspective view of a cartridge of an analytical kit according to a third embodiment of the present invention.
Fig. 24 includes a perspective view showing an example of informative portion of a cartridge.
Fig. 25 includes a sectional view showing the entirety of a cartridge and a principal portion of an analyzer in a state in which the cartridge is mounted to the analyzer in the analytical kit according to the third embodiment of the present invention.
Fig. 26 is an overall perspective view of a second member of a housing of the analyzer shown in Fig. 25.
Fig. 27 includes a sectional view of a principal portion of an analyzer according to a fourth embodiment of the present invention.
Fig. 28 is a sectional view of a principal portion of another example of analyzer.
Fig. 29 includes a sectional view of a principal portion of another example of analyzer.
Fig. 30 is a sectional view of a principal portion of another example of analyzer.

### BEST MODE FOR CARRYING OUT THE INVENTION

A first through a fourth embodiments of the present invention will be described below with reference to the accompanying drawings.

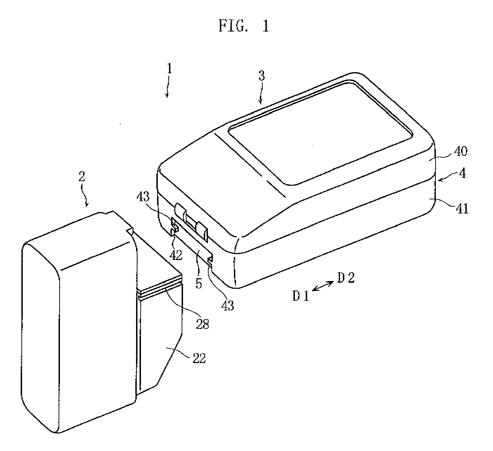

A first embodiment of the present invention will first be described with reference to Figs. 1-12.

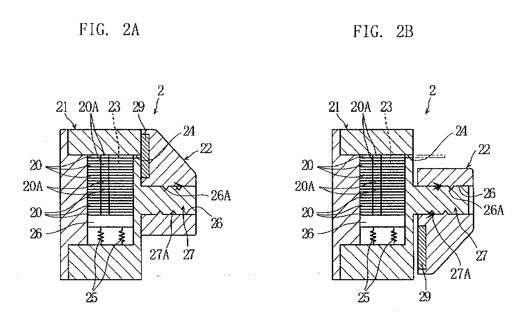

The analytical kit 1 shown in Fig 1 is so structured that an analytical instrument 20 is taken out of a cartridge 2 to perform sample analysis at an analyzer 3 using the analytical instrument 20 (See Figs. 2A and 2B).

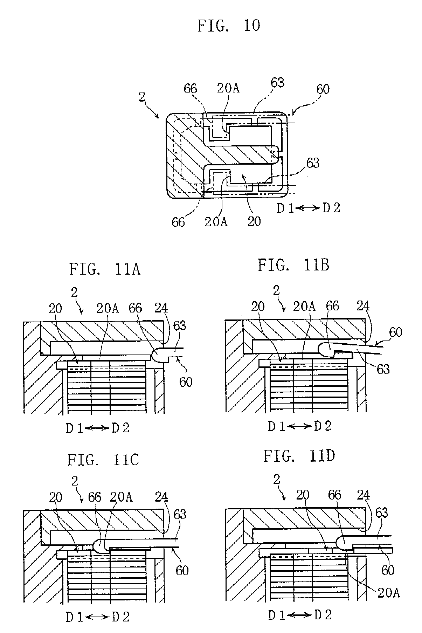

The cartridge 2 accommodates a plurality of analytical instruments 20 and is so designed that each of the analytical instruments 20 can be taken out by the analyzer 3. The analytical instruments 20 to be stored in the cartridge 2 are those for performing sample analysis by an optical or electrochemical method by using a small amount, e.g. about 0.1 to 3 µL of sample (e.g. blood or urine). Each of the analytical instruments 20 is in the form of a plate as a whole and formed with a pair of cutouts 20A, as shown in Figs. 2 and 10. Each of the cutouts 20A is provided for coming into engagement with an arm 63 of a movable member 60 of the analyzer 3 (See Fig. 10), which will be described later.

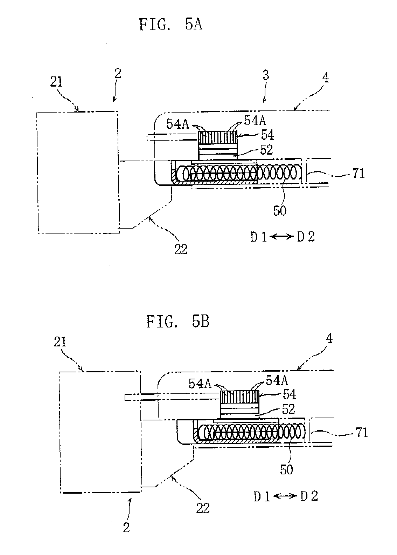

The cartridge 2, which is mounted in use to the analyzer 3 (See Figs. 5A and 5B) includes a main body 21 and a rotational member 22.

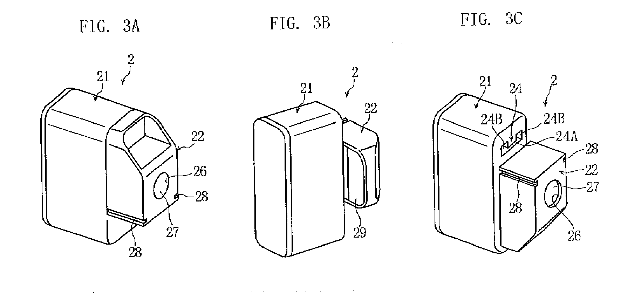

As shown in Figs. 2A and 2B, the main body 21 accommodates the analytical instruments 20 as stacked in the thickness direction of the analytical instruments and includes an accommodation space 23 and a discharge port 24. In the accommodation space 23, a support plate 26 connected to coil springs 25 is arranged. Thus, the analytical instruments 20 are stacked on the support plate 26 and biased upward by the coil springs 25. The discharge port 24 is utilized for taking out the analytical instruments 20 from the accommodation space 23. As shown in Fig. 3C, the discharge port 24 includes a thin portion 24A having a width corresponding to the thickness of a single analytical instrument 20, and a pair of thick portions 24B for allowing the insertion of arms 63 of the movable member 60 of the analyzer 3, which will be described later.

As shown in Figs. 2A, 2B and 3A-3C, the rotational member 22 is utilized for selectively making the discharge port 24 of the main body 21 exposed or unexposed (covered) and rotatably connected to the main body 21. Specifically, as better shown in Figs. 2A and 2B, the rotational member 22 is connected to the main body 21 by inserting a shaft 27 provided at the main body 21 into a through-hole 26. Though not clearly shown in the figures, the through-hole 26 is formed with a helical projection 26A containing only a single turn of helix. Correspondingly, the shaft 27 is formed with a helical groove 27A to mesh with the helical projection 26A. With this structure, the rotational member 22 is rotatable relative to the main body 21, with the projection 26A meshing with the groove 27A, and the rotational angle is controlled to 180 degrees. As shown in Figs. 3A and 3C, the rotational member 22 further includes a pair of grooves 28 extending in the axial direction of the through-hole 26, and a sealing member 29. The paired grooves 28 engage a pair of projections 43 at an opening 42 of the analyzer 3 in mounting the cartridge 2 to the analyzer 3. By bringing the projections 43 into engagement with the grooves 28 inmounting the cartridge 2 to the analyzer 3, the movement direction and position of the cartridge 2 relative to the analyzer 3 are controlled. The sealing member 29 serves to shield the discharge port 24 when the discharge port 24 is covered by the rotational member 22. The sealing member 29 is made of an elastic material such as rubber. By the provision of the sealing member 29, the discharge port 24 can be completely closed when the analytical instruments 20 are not to be taken out from the cartridge 2. Therefore, moisture and light are prevented from entering through the discharge port 24, whereby the analytical instrument 20 is prevented from deteriorating.

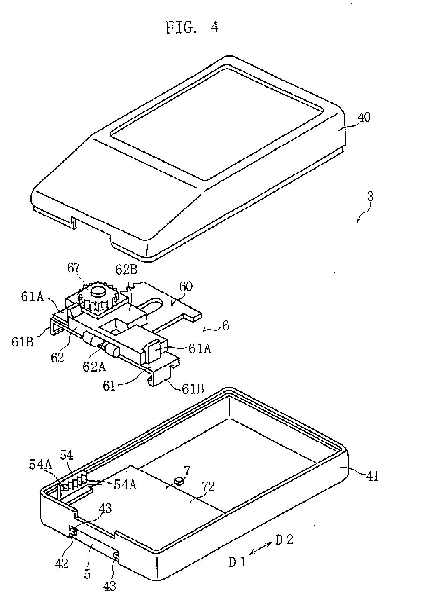

As shown in Figs. 1 and 4, the analyzer 3, which is utilized for performing sample analysis using the analytical instrument 20, includes a housing 4, an operational member 5, an extracting mechanism 6, and a fixation plate 7.

The housing 4 defines the appearance of the analyzer 3 and accommodates various parts such as the operational member 5 and the extracting mechanism 6. The housing 4 is made by combining a first and a second members 40 and 41 together so as to be hollow and includes an opening 42 for exposing the operational member 5 outside the housing 4. The paired projections 43 projecting inward are formed at the opening 42. In mounting the cartridge 2 to the analyzer 3, the projections 43 are brought into engagement with the grooves 28 of the rotational member 22 of the cartridge 2.

As shown in Figs. 4-8, the operational member 5, which is operated to move the movable member 60 of the extracting mechanism 6, is reciprocally movable relative to the housing 4 in the directions of D1, D2 while being partially exposed through the opening 42. The operational member 5 is biased in the direction of D1 by a pair of coil springs 50 so that the operational member moves in the direction of D2 when a load in the direction of D2 is applied thereto and returns to its wait position when the load in the direction of D2 is removed. The operational member 5 includes a pair of rails 51, a pair of guide members 52, 53 and a rack portion 54.

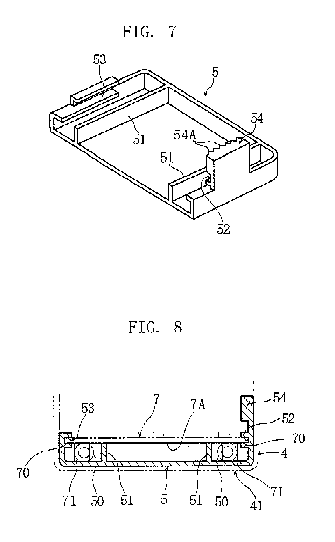

As better shown in Fig. 8, the paired rails 51 and guide members 52, 53 are brought into engagement with the fixation plate 7 and enable the operational member 5 to move as desired in a space defined between the fixation plate 7 and the second member 41 of the housing 4. Specifically, the paired rails 51 are held in contact with the bottom surface 7A of the fixation plate 7, whereas each of the paired guide members 52 and 53 holds therein a side edge 70 of the fixation plate 7. The rack portion 54 is used for transmitting motive power to a rack portion 64 of the movable member 60, which will be described later, and includes a plurality of cogs 54A (See Figs. 4 and 5).

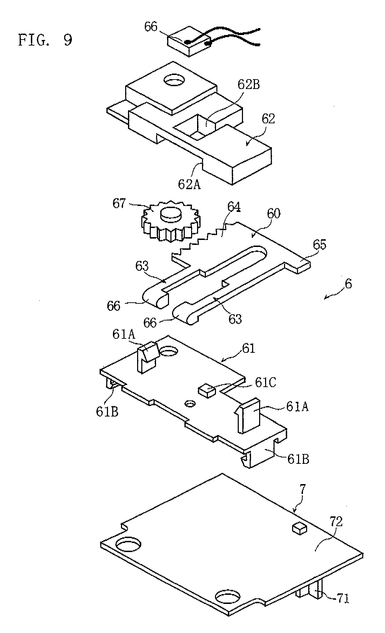

As shown in Figs. 4 and 9, the extracting mechanism 6 serves to take the analytical instruments 20 out of the cartridge 2 and is made up of the movable member 60, a first supporting member 61 and a second supporting member 62.

The movable member 60 moves in the directions of D1, D2 in accordance with the movement of the operational member 5 and includes a pair of arms 63, a rack portion 64 and a detection projection65. The paired arms 63 a re the port ionstobe inserted into the accommodation space 23 of the cartridge 2 to engage with the analytical instruments 20 and take out the analytical instruments 20. Each of the arms 63 includes a hook 66 for coming into engagement with the cutout 20A of the analytical instrument 20. The hook 66 includes a rounded surface on the direction D1 side. The rack portion 64 is utilized for inputting a load for moving the movable member 60 and is connected to the rack portion 54 of the operational member 5 via a gear 67. The detection projection 65 serves to turn on a switch 72, which will be described later.

The first and the second supporting members 61 and 62 serve to define the movement path of the movable member 60 and connect the movable member 60 to the fixation plate 7 so as to be movable relative to the fixing plate. The first supporting member 61 includes hooks 61A and 61B for fixing the first supporting member 61 to the second supporting member 62 and the fixation plate 7. The first supporting member 61 further includes a stopper 61C. When the movable member 60 is moved in the direction of D2 while holding the analytical instrument 20, the stopper 61C comes into contact with an end of the analytical instrument 20. In this way, the stopper 61C serves to restrict the movement of the analytical instrument 20 in the direction of D2 and locate the analytical instrument 20 at a target position (applying position). When the movable member 60 moves, the stopper 61C is positioned between the paired arms 63 and does not hinder the movement of the movable member 60. The second supporting member 62 includes a recess 62A and a through-hole 62B. The recess 62A defines a space for allowing the movement of the movable member 60 when the second supporting member 62 is fixed to the first supporting member 61. The through-hole 62B is utilized for arranging a detection mechanism 66 such as a photometry mechanism for obtaining information as to the sample from the analytical instrument 20.

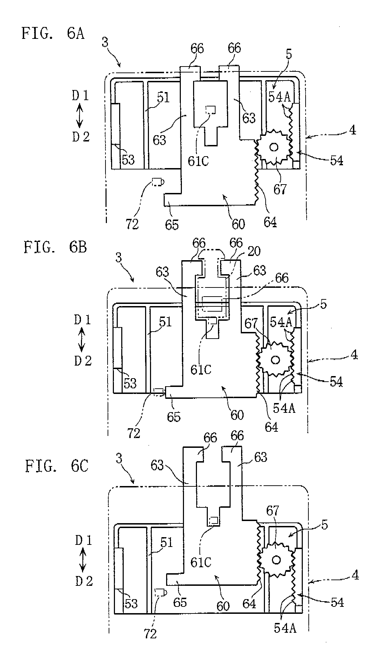

The gear 67 is rotatably fixed between the first and the second supporting members 61 and 62. As shown in Figs. 6A-6C, the gear 67 is fixed at a position where the gear can engage both of the rack portion 64 of the movable member 60 and the rack portion 54 of the operational member 5. Thus, when the operational member 5 is moved, the gear 67 rotates and transmits the rotational force to the movable member 60 to move the movable member 60 in a direction opposite to the movement direction of the operational member 5. Specifically, when a load in the direction of D2 is applied to the operational member 5, the movable member 60 moves in the direction of D1. As a result, the arms 63 project largely from the housing 4 so as to be inserted into the main body 21 of the cartridge 2. Conversely, when the load on the operational member 5 is removed and the operational member 5 moves in the direction of D1, the movable member 60 moves in the direction of D2.

As shown in Figs. 6A-6C and Fig. 8, the fixation plate 7 fixes the extracting mechanism 6 to the housing 4 (second member 41) and define, together with the second member 41, the movement path of the operational member 5. The fixation plate 7 is fixed to the second member 41 and includes a pair of legs 71. The paired legs 71 define the distance between the second member 41 and the fixation plate 7 and are held in engagement with respective one ends of the coil springs 50 (See Fig. 5).

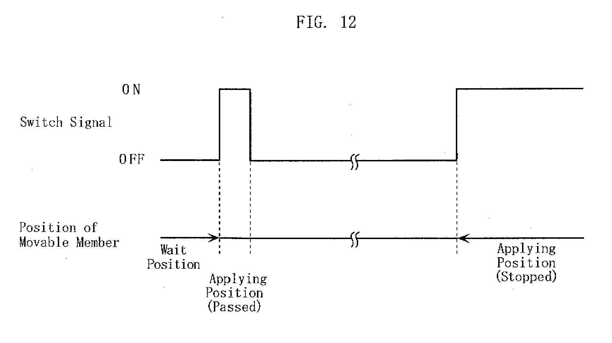

The fixation plate 7 is provided with a switch 72. The switch 72 is turned on and off by the detection projection 65 of the movable member 60 and so positioned as to be turned on by the detection projection 65 when the movable member 60 is located at the applying position. Specifically, as shown in Fig. 12, when the switch 72 is off in the initial state, the movable member 62 is found to be positioned at the wait position (i.e., the state in which no load is exerted on the operational member 5 (i.e. the state in which the cartridge 2 is not mounted to the analyzer 3)). In the period from the initial state until the switch 72 is turned on, the movable member 60 moves from the wait position to the applying position (the position which is set between the wait position and a take-out position and where the applying of a sample to the analytical instrument 20 and the sample analysis are performed). Thereafter, when the switch 72 is turned on and the on-state is maintained, the movable member 60 is found to be positioned at the applying position while holding the analytical instrument 20. That is, when the movable member 60 is caused to hold the analytical instrument 20 and moved in the direction of D2, the analytical instrument 20 comes into contact with the stopper 61C, whereby the movement of the movable member 60 is restricted. The position of the movable member 60 in this state corresponds to the position at which the detection projection 65 turns on the switch 72. Therefore, when the on-state of the switch 72 is maintained, the movable member 60 is found to be located at the applying position while holding the analytical instrument 20.

The movable member 60 may be provided with a plurality of detection projections 65, and a plurality of switches 72 may be provided so that the position of the movable member 60 can be detected by the combination of ON/OFF information obtained from the plurality of switches. In this case, it is possible to detect at least three positions, i.e., the wait position, the take-out position (the position of the movable member 60 when the operational member 5 is moved to the deepest portion in the direction of D2), and the applying position 3.

The method of sample analysis using the analytical kit 1, the usage of the analytical kit 1, and the operation of the cartridge 2 and the analyzer 3 will be described below.

First, to analyze a sample by using the analytical kit 1, an analytical instrument 20 accommodated in the cartridge 2 is fed to the analyzer 3. The feeding of the analytical instrument 20 to the analyzer 3 is performed by mounting the cartridge 2 to the analyzer 3 and then detaching the cartridge 2 from the analyzer 3.

The mounting of the cartridge 2 to the analyzer 3 is performed after the discharge port 24 is exposed by the user by manually rotating the rotational member 22 of the cartridge 2 through 180 degrees, as shown in Figs. 3A-3C. In this state, as shown in Figs. 1 and 5, the grooves 28 of the rotational member 22 are positioned relative to the projections 43 of the housing 4, and a force in the direction of D2 is applied to the operational member 5 via the rotational member 22 of the cartridge 2. As a result, as shown in Figs. 6A-6C, the movable member 60 moves in the opposite direction (D1 direction) from the operational member 5, so that the arms 63 of the movable member 60 enter the cartridge 2 through the discharge port 24, as shown in Figs. 10 and 11A-11C. Since the hook 66 of each of the arms 63 has a rounded shape, the arm 63 rides on the upper surface of the uppermost one of the analytical instruments 20 accommodated in the cartridge 2. Then, the hook 66 falls into the cutout 20A of the analytical instrument 20 to engage the cutout 20A.

The detachment of the cartridge 2 from the analyzer 3 is performed by the user by moving the cartridge 2 relative to the analyzer 3 in the direction of D1. By this operation, the load which has been applied to the operational member 5 in the direction of D2 is removed, so that the operational member 5 moves in the direction of D1, while the movable member 60 moves in the direction of D2 (See Figs. 6A-6C). Since the hooks 66 of the movable member 60 are held in engagement with the cutouts 20A of the analytical instrument 20, the analytical instrument 20 moves together with the movable member 60 in the direction of D2 relative to the cartridge 2 and is thereby taken out of the cartridge 2, as shown in Figs. 11C and 11D. As shown in Fig. 6B, when the movable member 60 holding the analytical instrument 20 is moved in the direction of D2, the stopper 61C restricts the movement of the analytical instrument 20 (movable member 60) in the direction of D2. As a result, the analytical instrument 20 is so located that an end thereof is exposed out of the analyzer 3, while a portion within the analyzer 3 faces the detection mechanism 66.

In this state, the switch 72 is continuously kept "ON", so that it is detected in the analyzer 3 that the movable member 60 is located at the applying position while holding the analytical instrument 20. In the case where it is detected in the analyzer 3 that the analytical instrument 20 (movable member 60) is located at the applying position, the analyzer 3 actuates the detection mechanism 66 after the lapse of a predetermined time period from the detection or when the user operates a button to notify that the applying of the sample is completed. For instance, in the case where the analytical instrument is designed to analyze a sample by an optical method, the detection mechanism 66 is structured as a photometry mechanism. In this case, light is directed to the analytical instrument 20, and the reflected light is received. Based on the received amount of light, computation necessary for the sample analysis is performed in the analyzer 3. Alternatively, the photometry mechanism may be designed to receive the transmitted light. The analytical instrument 20 and the detection mechanism 66 may be designed to perform sample analysis by an electrochemical method.

After the sample analysis is finished, the analytical instrument 20 is disposed of. The disposal of the analytical instrument 20 can be performed by the user by moving the operational member 5 in the direction of arrow D2. Specifically, when the operational member 5 is moved in the direction of D2, the movable member 60 moves in the direction of D1, whereby the analytical instrument 20 also moves in the direction of D1. When the movable member 60 is moved to the take-out position, the analytical instrument 20 entirely projects out of the housing 4 and drops by its own weight. In this way, the analytical instrument 20 is removed from the analyzer 3.

In the analytical kit 1 according to the present invention, an analytical instrument 20 is fed to the analyzer 3 by mounting the cartridge 2 to the analyzer 3 and then detaching the cartridge 2 from the analyzer 3. Therefore, the user does not need to insert the analytical instrument 20 into the analyzer 3 nor take the analytical instrument 20 out of the container or package and can feed the analytical instrument 20 to the analyzer 3 by an extremely simple operation. Further, the analytical instrument 20 after the sample analysis can be disposed of by the user by operating the operational member 5. Therefore, the user can remove the analytical instrument hygienically by an extremely simple operation without touching the analytical instrument 20 to which the sample such as blood adheres. Moreover, the portions of the analyzer 3 and the cartridge 2 which are related to the taking-out and disposal of the analytical instrument 20 have a simple structure and can be structured inexpensively. Therefore, the reduction of burden on the user in the analysis operation can be achieved without considerably increasing the apparatus cost and the analysis cost.

The movable member 60 can stop at the applying position only when it holds the analytical instrument 20, and the fact that the movable member 60 is located at the applying position can be detected based on the output from the switch 72. Therefore, the sample analysis operation is prevented from being performed in a state in which the analytical instrument 20 is not fed to the analyzer 3.

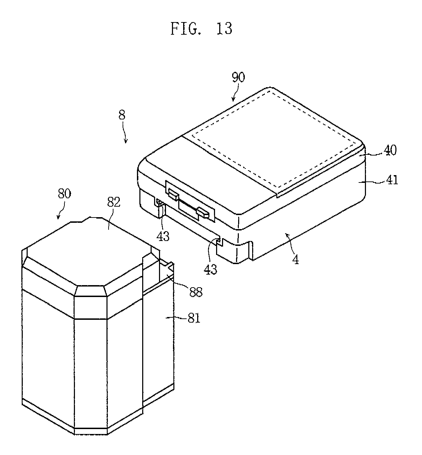

A second embodiment of the present invention will be described below with reference to Figs. 13-22.

The analytical kit 8 shown in Fig. 13 includes a cartridge 80 and an analyzer 90. The principle of taking out the analytical instrument 20' is basically similar to that of the analytical kit 1 (See Fig. 1) of the first embodiment.

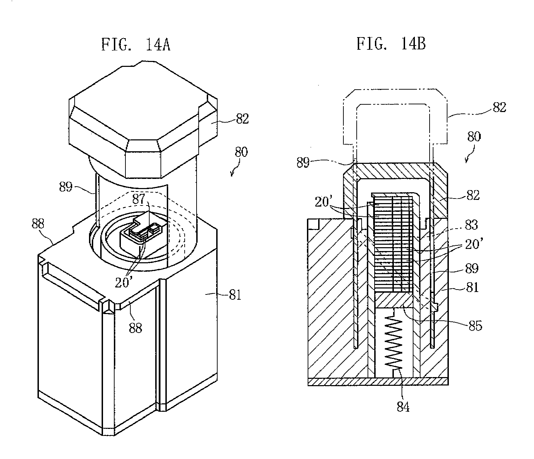

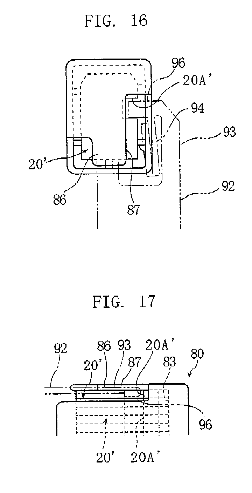

As shown in Figs. 14A and 14B, the cartridge 80 accommodates a plurality of analytical instruments 20', each of which can be taken out by the analyzer 90 (See Fig. 13), The analytical instruments 20' stored in the cartridge 80 are similar to the analytical instruments 20 of the first embodiment. Unlike the first embodiment, however, each of the analytical instruments 20' includes only a single cutout 20A' provided for engagement with an arm 93 of a movable member 92 of the analyzer 90, which will be described later (See Fig. 16). The cartridge 80 includes a main body 81 and a rotational member 82, and the rotational member 82 is movable vertically relative to the main body 81.

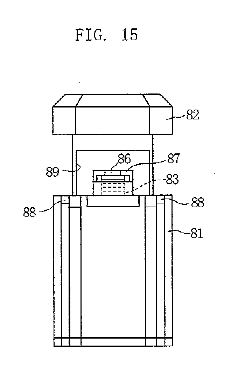

The main body 81 accommodates the analytical instruments 20' as stacked in the thickness direction (vertical direction) of the analytical instruments and includes an accommodation space 83. In the accommodation space 83, a support plate 85 connected to a coil spring 84 is arranged. Thus, the analytical instruments 20' are stacked on the support plate 85 and biased upward by the coil spring 84. As shown in Figs. 15-17, the top position of the accommodation space 83 is defined by a wall 86. The uppermost one of the analytical instruments 20' which are biased upward is held in engagement with the wall 86. The wall 86 is formed with a cutout 87 for coming into contact with an arm 93 of a movable member 92, which will be described later.

The main body 81 further includes a pair of guide flanges 88. In mounting the cartridge 80 to the analyzer 90, the paired guide flanges 88 are brought into engagement with a pair of recesses 43 (See Fig. 13) of the housing 4 of the analyzer 90. Thus, by bringing the guide flanges 88 into engagement with the recesses 43 in mounting the cartridge 80 to the analyzer 90, the movement direction and position of the cartridge 80 relative to the analyzer 90 are controlled.

As shown in Figs. 14A, 14B and 15, the rotational member 82 includes a discharge port 89 and makes it possible to select a state in which the analytical instruments 20' can be taken out from the main body 81 or a state in which the analytical instruments cannot be taken out. The rotational member 82 is rotatably screwed to the main body 81, and the rotational angle is controlled to 180 degrees. The applying pot 89 is utilized for taking the analytical instrument 20' out of the accommodation space 83 and allows the insertion of the arm 93 of the movable member 92. The position of the discharge port 89 changes by rotating the rotational member 82. Specifically, since the rotational member 82 slides vertically by its rotation, the position of the discharge port 89 changes in accordance with the rotation and the vertical sliding of the rotational member 82. Instead of the rotational member 82, a member which just slides vertically may be employed as the selector. In this case, the position of the discharge port changes only in the vertical direction.

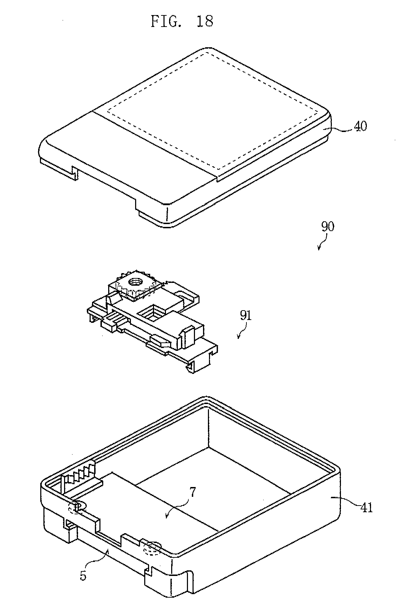

As shown in Figs. 13 and 18, the analyzer 90, which performs sample analysis using the analytical instrument 20', includes a housing 40, 41 (4), an operational member 5, an extracting mechanism 91, and a fixation plate 7, similarly to the foregoing analyzer 3 (See Fig. 4).

The housing 4 and the operational member 5 are basically similar to the housing 4 and the operational member 5 (See Fig. 4) of the foregoing analyzer 3, although the form is slightly different. In the figures, the portions which function similarly to the foregoing housing 4 and the operational member 5 (See Fig. 4) are indicated by the same reference signs.

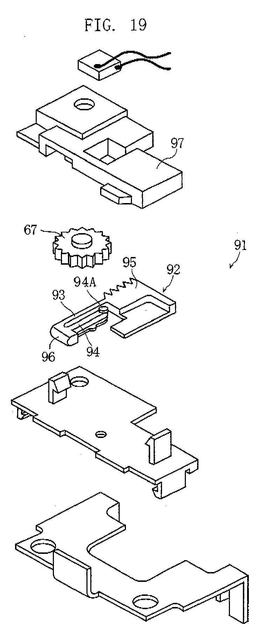

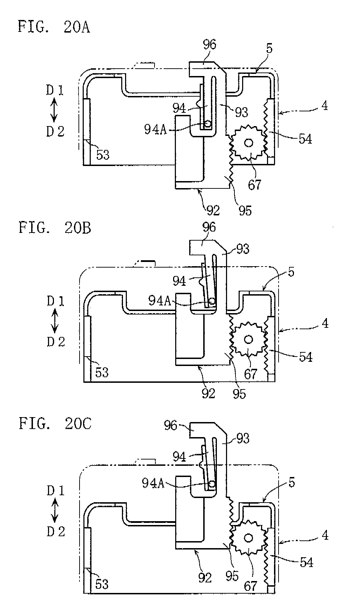

As shown in Figs. 18 and 19, the extracting mechanism 91 serves to take the analytical instruments 20' out of the cartridge 80. As shown in Figs. 20A-20C, similarly to the extracting mechanism 6 of the first embodiment (See Figs. 4 and 9), the extracting mechanism 91 is so designed that the movable member 92 moves in the direction of D1 to cause the arm 93 project largely from the housing 4 when a load in the direction of D2 is applied to the operational member 5, while the movable member 92 moves in the direction of D2 when the load on the operational member 5 is removed and the operational member 5 moves in the direction of D1. In the figures, the elements which are identical or similar to those of the extracting mechanism 6 of the first embodiment (See Figs. 4 and 9) are designated by the same reference signs as those used for the first embodiment.

The movable member 92 moves in the directions of D1, D2 in accordance with the movement of the operational member 5 and includes a single arm 93, a pivotable portion 94 and a rack portion 95. Thus, the movable member 92 differs from the movable member 60 of the first embodiment (See Figs. 6 and 9) in that only a single arm 93 is provided and that the pivotable portion 94 is provided.

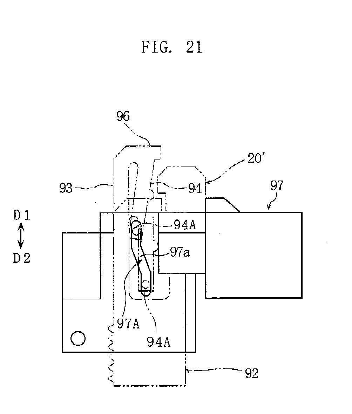

As shown in Figs. 16, 17 and 20, the arm 93 is the portion to be inserted into the accommodation space 83 of the cartridge 80 to engage with the analytical instruments 20' and take out the analytical instruments 20'. The arm 93 includes a hook 96 for coming into engagement with the cutout 20A' of the analytical instrument 20'. The hook 96 includes a rounded surface on the direction D1 side. The pivotable portion 94 extends from the hook 96 in the direction of D2 and is pivotable in accordance with the movement of the movable member 92. The pivotable portion 94 includes a projection 94A projecting upward. The projection 94A is provided for coming into engagement with a groove 97A (See Fig. 21) of a second supporting member 97, which will be described later. Specifically, when the movable member 92 moves as shown in Fig. 20, the pivotable portion 94 pivots due to the movement of the projection 94A within the groove 97A. The rack portion 95 is utilized for inputting a load for moving the movable member 92 and is connected to the rack portion 54 of the operational member 5 via a gear 67, which will be described later.

As shown in Fig. 21, the second supporting member 97 is formed with the groove 97A for engagement with the projection 94A of the pivotable portion 94 of the movable member 92. The groove 97A includes an inclined groove portion 97a which is inclined with respect to the directions of D1, D2. When the projection 94A moves along the inclined groove portion 97a, the pivotable portion 94 pivots. The inclined groove portion 97a is so designed that the projection 94A approaches the arm 93 when the movable member 92 (arm 93) moves in the direction of D1, while the projection 94A moves away from the arm 93 when the movable member 92 (arm 93) moves in the direction of D2.

In the above-described analytical kit 8, the method of sample analysis, the usage of the analytical kit 8, and the operation of the cartridge 80 and the analyzer 90 are basically similar to those of the analytical kit 1 (See Fig. 1) of the first embodiment.

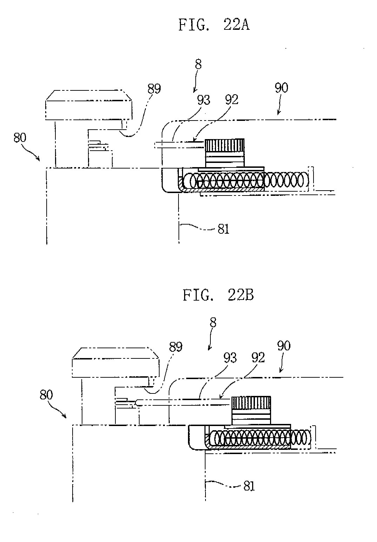

Specifically, to analyze a sample by using the analytical kit 8, an analytical instrument 20' accommodated in the cartridge 80 is fed to the analyzer 90 by mounting the cartridge 80 to the analyzer 90 and then detaching the cartridge 80 from the analyzer 90, as shown in Figs. 22A and 22B.

As shown in Figs. 13, 14A and 14B, to mount the cartridge 80 to the analyzer 90, the rotational member 82 of the cartridge 80 is rotated manually by the user through 180 degrees so that the discharge port 89 is oriented in the direction of D2 in mounting the cartridge 80 to the analyzer 90. In this state, the guide flanges 88 of the main body 81 are positioned relative to the recesses 43 of the housing 4, and a force in the direction of D2 is applied to the operational member 5 via the main body 81 of the cartridge 80, as shown in Figs. 22A and 22B. As a result, as shown in Figs. 20A-20C, the movable member 92 moves in the opposite direction (D1 direction) from the operational member 5, so that the arm 93 of the movable member 92 enters the cartridge 80 through the discharge port 89, as shown in Figs. 22A and 22B. Since the hook 96 of the arm 93 has a rounded shape as shown in Fig. 17, the arm rides on the upper surface of the uppermost one of the analytical instruments' 20 accommodated in the cartridge 80. Then, the hook 96 falls into the cutout 20A' of the analytical instrument 20' to engage the cutout 20A'. (The movement of the hook 96 to engage the cutout 20A' is similar to that of the first embodiment (See Figs. 11A-11C)). As shown in Fig. 21, the pivotable portion 94 of the movable member 92 approaches the arm 93 due to the movement of the projection 94A along the inclined groove portion 97a.

The detachment of the cartridge 80 from the analyzer 90 is performed by the user by moving the cartridge 80 relative to the analyzer 90 in the direction of D1. By this operation, the load which has been applied to the operational member 5 in the direction of D2 is removed, so that the operational member 5 moves in the direction of D1, while the movable member 92 moves in the direction of D2 (See Figs. 20A-20C). Since the hook 96 of the movable member 92 is held in engagement with the cutout 20A' of the analytical instrument 20', the analytical instrument 20' moves together with the movable member 92 in the direction of D2 relative to the cartridge 80 and is thereby taken out of the cartridge 80. The pivotal portion 94 of the movable member 92 moves away from the arm 93 due to the movement of the projection 94A along the inclined groove portion 97a in the direction opposite to the above. As a result, a load in the direction to be away from the arm 93 is applied to the analytical instrument 20', so that the analytical instrument 20' can have an appropriate posture.

The analysis of the sample and the disposal of the analytical instrument 20' are performed similarly to the first embodiment.

With the above-described analytical kit 8, the feeding of the analytical instrument 20' to the analyzer 90 and the disposal of the analytical instrument 20' from the analyzer 90 can be performed without imposing any burden on the user and with a simple and inexpensive structure.

The analyzer 3, 90 of the first and the second embodiments are so structured that the analytical instrument 20, 20' are taken out by applying a force in the direction of D2 to the operational member 5 by the cartridge 80. However, the analyzer may be so structured that the analytical instrument 20, 20' is taken out of the cartridge 2, 80 by operating the operational member 5 by the user.

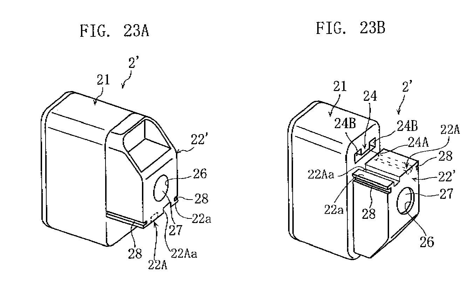

A third embodiment of the present invention will be described below with reference to Figs. 23-25. ln these figures, the elements or portions which are identical or similar to those of the cartridge 2 and the analyzer 3 of the first embodiment are designated by the same reference signs as those used for the first embodiment, and the overlapping description is omitted.

The cartridge 2' shown in Figs. 23A and 23B is similar in basic structure to the cartridge 2 of the analytical kit 1 of the first embodiment (See Figs. 1-3) but differs from the cartridge 2 in structure of the rotational member 22'.

The rotational member 22' includes an informative portion 22A for giving information on the analytical instrument 20' to the analyzer 3'. The informative portion 22A is provided at a side surface 22a of the rotational member 22' and causes the analyzer 3' to recognize the intended information by selectively including a groove 22Aa at predetermined regions of the side surface 22a. As better shown in Fig. 23B, the side surface 22a is a surface which is oriented upward when the discharge port 24 of the main body 21 is exposed for use.

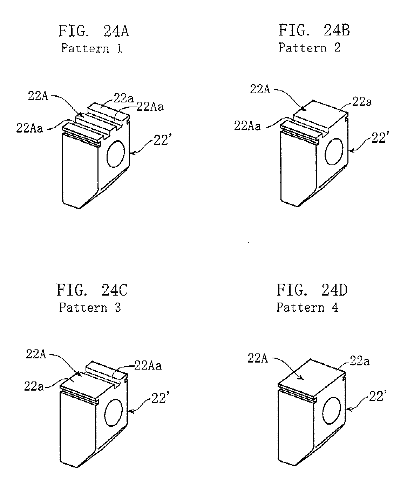

When the number of the predetermined regions are two, the informative portion 22A can select an appropriate one from four patterns, i.e., the pattern in which a groove 22Aa is provided at each of the two regions as shown in Fig. 24A (pattern 1), the patterns in which a groove 22Aa is provided at either one of the two regions as shown in Figs. 24B and 24C (pattern 2 and pattern 3) and the pattern in which a groove is not provided as shown in Fig. 24D. Thus, when two regions are predetermined as shown in Figs. 24A-24D, four kinds of information can be outputted.

The information to be applied to the rotational member 22' (informative portion 22A) is information related to the analytical instrument 20, and typically information as to the sensitivity of the analytical instrument 20. Other examples of information to be applied to the informative portion 22A include the manufacture country, manufacture factory, manufacture line, manufacture date and specification.

In the informative portion 22A, the number of regions where a groove is selectively formed may be one or more than two. Further, instead of a groove, the informative portion 22A may selectively include a projection or a recess other than a groove.

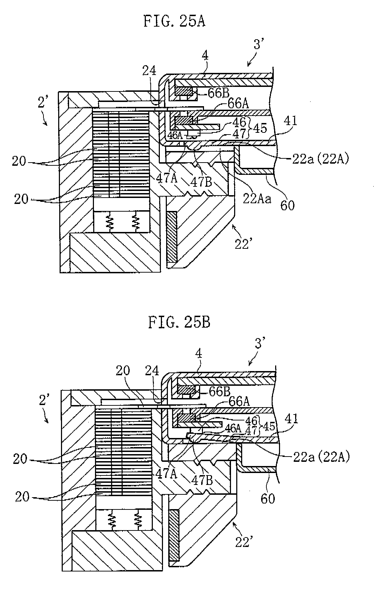

The analyzer 3' shown in Figs. 25A and 25B is similar in basic structure to the analyzer 3 (See Figs. 4-11) of the analytical kit 1 of the first embodiment but differs from the analyzer 3 in that the analyzer 3' includes an information recognizer 45.

The information recognizer 45 includes a pair of switches 46 and a pair of leaf springs 47 provided correspondingly to the pair of switches 46.

Each of the switches 46 is provided with a downwardly projecting button 46A and outputs an ON signal when the button 46A is pressed.

Each of the leaf springs 47 is provided at the second member 41 of the housing 4 and serves to selectively press the button 46A. The entirety of the leaf spring 47 has the ability of spring so that the end 47A thereof can move up and down. The end 47A of the leaf spring 47 is provided with a downward projection 46B. The projection 46B is capable of coming into contact with the side surface 22a of the rotational member 22' of the cartridge 2' when the cartridge 2' is mounted to the analyzer 3'. The projection 46B is provided at a position corresponding to the predetermined region (informative portion 22A) of the side surface 22a at which the groove 22Aa is selectively formed. Therefore, as shown in Fig. 25A, in the case where the informative portion 22A includes a groove 22Aa, the projection 47B of the leaf spring 47 is received in the groove 22Aa of the rotational member 22' when the cartridge 2' is mounted to the analyzer 3'. Therefore, the leaf spring 47 keeps its original state so that the button 46A of the switch 46 is not pressed. On the other hand, as shown in Fig. 25B, in the case where the informative portion 22A does not include a groove 22Aa, the projection 47B of the leaf spring 47 engages the side surface 22a of the rotational member 22' when the cartridge 2' is mounted to the analyzer 3'. As a result, the end 47A of the leaf spring 47 is pushed upward and presses the button 46A of the switch 46.

As described above, as the informative portion 22A of the rotational member 22' of the cartridge 2', two regions are predetermined at each of which a groove 22Aa is to be formed selectively. Thus, by selecting whether or not a groove 22Aa is to be formed at each of the regions, suitable one selected from four kinds of outputs (information) can be outputted. That is, the paired switches 46 can output the combinations of signals as given in Table 1 below.

**Table 1**

| pattern of informative portion | Paired Switch ON/OFF | |
|---|---|---|
| Pattern 1 | ON | ON |
| Pattern 2 | ON | OFF |
| Pattern 3 | OFF | ON |
| Pattern 4 | OFF | OFF |

The operation and advantages of the analytical kit as the combination of the cartridge 2' and the analyzer 3' will be described below.

To perform analysis using the analytical kit, the rotational member 22' of the cartridge 2' is first rotated to expose the discharge port 24 of the cartridge 2', as shown in Figs. 23A and 23B. In this state, the side surface 22a of the rotational member 22', i.e., the informative portion 22A is oriented upward.

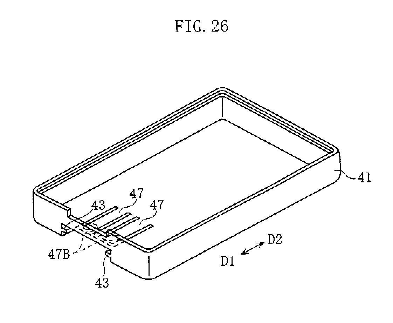

Subsequently, with the grooves 28 (See Fig. 23B) of the rotational member 22 positioned relative to the projections 43 (See Figs. 1 and 26) of the housing 4, the cartridge 2' is mounted to the analyzer 3', as shown in Figs. 25A and 25B. At this time, though not illustrated in the figures, the arms 63 of the movable member 60 enter the cartridge 2 through the discharge port 24, and the hooks 66 engage the cutouts 20A of the analytical instrument 20 (See Figs. 6A-6C, 10 and 11A-11C).

At the information recognizer 45 of the analyzer 3', each of the paired switches 46 selectively outputs an ON signal. Specifically, as described above, the button 46A of the switch 46 is not pressed when the informative portion 22A includes a groove 22Aa as shown in Fig. 25A, whereas the button 46A of the switch 46 is pressed when the informative portion 22A does not include a groove 22Aa as shown in Fig. 25B. In this way, any of the combinations of signals as given in Table 1 is outputted from the paired switches 46.

Subsequently, the cartridge 2' is detached from the analyzer 3'. As a result, the operational member 5 moves in the direction of D1, whereas the movable member 60 moves in the direction of D2 (See Figs. 6A-6C). Therefore, the analytical instrument 20 moves together with the movable member 60 in the direction of D2 relative to the cartridge 2' and is thereby taken out of the cartridge 2' (See Figs. 11C and 11D).

In the analyzer 3', after the lapse of a predetermined time period or when the user operates a button to notify that the applying of the sample is completed, the detection mechanism 66A, 66B is actuated to perform the sample analysis. The detection mechanism 66A, 66B comprises a light emitting portion 66A and a light receiving portion 66B. The light emitting portion 66A directs light to the analytical instrument 20, whereas the light receiving portion 66B receives the reflected light. In the analyzer 3', based on the received amount of light, computation necessary for the sample analysis is performed.

The sample analysis is performed by applying the output from the light receiving portion 66B to a predetermined calibration curve. Specifically, a plurality of calibration curves are prepared in advance, and the calibration curve to be used is selected based on the combination of outputs from the paired switches 46 which is recognized by the information recognizer 45. Thus, in the analyzer 3', a calibration curve corresponding to the sensitivity of the analytical instrument 20 is selected, and the sample analysis is performed based on the calibration curve.

The disposal of the analytical instrument 20 is performed similarly to the first embodiment.

With the cartridge 2' and the analyzer 3' of the present invention, the sample analysis is performed based on a calibration curve corresponding to the sensitivity of the analytical instrument 20. Therefore, variation in the analysis accuracy due to the variation in sensitivity of the analytical instrument 20 can be prevented, whereby the analysis accuracy can be enhanced.

This advantage is obtained just by providing the informative portion 22A (groove 22Aa) at the rotational member 22' of the cartridge 2' while providing the information recognizer 45 of an extremely simple structure at the analyzer 3'. Further, the information as to the analytical instrument 20 such as the sensitivity of the analytical instrument 20 can be obtained in the process required for the sample analysis such as the process of mounting the cartridge 2' to the analyzer 3'. Therefore, any additional burden is not imposed on the user.

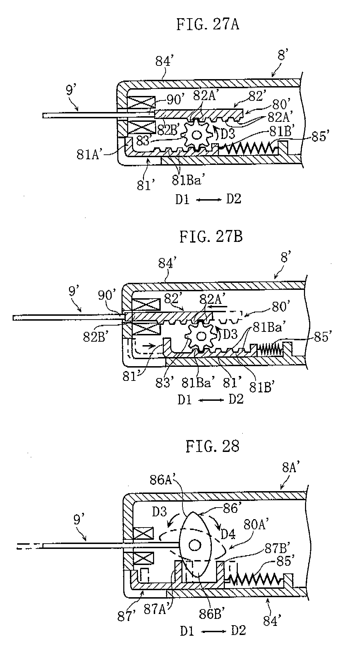

With reference to Figs. 27A and 27B, a fourth embodiment of the present invention will be described.

The analyzer 8' shown in the figures is characterized in that it includes a disposal mechanism 80' for disposing of an analytical instrument 9'.

The disposal mechanism 80' includes an operational member 81', a movable member 82' and a gear 83'.

The operational member 81' is reciprocally movable in the directions of D1, D2 relative to the housing 84' and includes an input end 81A' and a rack portion 81B' provided with a plurality of cogs 81Ba'. The operational member 81' is connected to the housing 84' via a spring 85' and biased by the spring 85' in the direction of D1. Thus, the operational member 81' moves in the direction of D2 when a load in the direction of D2 is inputted as shown in Fig. 27B and moves in the direction of D1 when the load in the direction of D2 is removed to return to the position shown in Fig. 27A.

The movable member 82' serves to push an end 90' of an analytical instrument 9' to move the analytical instrument 9' in the direction of D1. The movable member is reciprocally movable in the directions of D1, D2 relative to the housing 84'. The movable member 82' includes a plurality of cogs 82A' and moves in accordance with the movement of the operational member 81' in the opposite direction from the operational member 81'. The movable member 82' is so arranged that, in the wait state, i.e., when a load in the direction of D1 is not applied to the operational member 81', the end 82B' engages or faces the end 90' of the analytical instrument 9' mounted to the analyzer 8'.

The gear 83' functions as a link member for transmitting the load inputted to the operational member 81' to the movable member 82' and meshes with the cogs 81Ba of the operational member 81' and the cogs 82A' of the movable member 82'.

In the disposal mechanism 80', when a load is not applied to the operational member 81', the operational member 81' and the movable member 82' are located at the wait position, as shown in Fig. 27A. When a load in the direction of D2 is applied to the operational member 81' via the input end 81A', the operational member 81' moves in the direction of D2. In this movement, the gear 83' meshing with the cogs 81Ba' of the operational member 81' is rotated in the direction indicated by the arrow D3. Since the gear 83' meshes with the cogs 82A' of the movable member 82', the movable member 82' moves in the opposite direction from the operational member 81', i.e., in the direction of D1 in accordance with the rotation of the gear 83' in the direction of D3. As a result, the end 90' of the analytical instrument 9' is pushed by the end 82B' of the movable member 82' in the direction of D1, whereby the analytical instrument 9' moves in the direction of D1. As a result, the analytical instrument 9' becomes removable from the analyzer 8'. When a load on the operational member 81' in the direction of D2 is removed, the operational member 81' moves in the direction of D1 due to the elastic force of the spring 85' to return to the wait position. In accordance with this movement of the operational member 81', the movable member 82' moves in the direction of D2 to return to the wait position.

With the analyzer 8', by operating the disposal mechanism 80' directly above e.g. a trash can and making the analytical instrument 9' removable from the analyzer 8', the user can put the analytical instrument 9' after use into the trash can without touching the analytical instrument 9', which is hygienic. Moreover, the disposal of the analytical instrument 9' from the analyzer 8' can be performed by an extremely easy operation of applying a load to the operational member 81', which is convenient for the user.

The disposal mechanism of the analyzer 8' may be structured as shown in Figs. 28-30.

The disposal mechanism 80A' shown in Fig. 28 utilizes a rotational cam 86' as the movable member. The rotational cam 86' includes a cam surface 86A' for engagement with the end 90' of an analytical instrument 9'.

The operational member 87' includes a pair of engagement pieces 87A' and 87B' and is connected to the housing 84' via a spring 85'. The paired engagement pieces 87A' and 87B' are spaced from each other in the direction of D1, D2, and an end 86B' of the rotational cam 86' is arranged at the engagement pieces 87A' and 87B'. Specifically, when the operational member 87' is moved in the direction of D2, the engagement piece 87A' engages the end 86B' of the rotational cam 86' to rotate the rotational cam 86' in the direction of D3. When the operational member 87' is moved in the direction of D1, the engagement piece 87B' engages the end 86B' of the rotational cam 86' to rotate the rotational cam 86' in the direction of D4.

In the disposal mechanism 80A', when a load is not applied to the operational member 87', the operational member 87' and the rotational cam 86' are located at the wait position, as indicated by a solid line in Fig. 28. When a load in the direction of D2 is applied to the operational member 87', the operational member 87' moves in the direction of D2, as indicated by a double-dashed line in Fig. 28. When the operational member moves in this way, the rotational cam 86' rotates in the direction indicated by the arrow D3, so that the end 90' of the analytical instrument 9' moves along the cam surface A'. As a result, the analytical instrument 9' receives a load in the direction of D1 to become removable from the analyzer 8'. When the load applied to the operational member 87' in the direction of D2 is removed, the operational member 87' moves in the direction of D1 due to the elastic force of the spring 85' to return to the wait position. In accordance with this movement of the operational member 87', the rotational cam 86' rotates in the direction of D4 to return to the wait position.

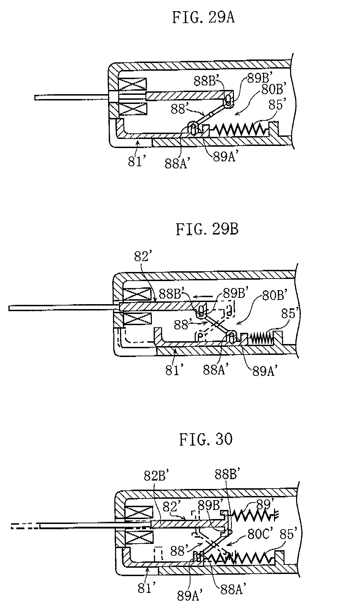

In the disposal mechanisms 80B' and 80C' shown in Figs. 29A, 29B and Fig. 30, as the link member for connecting the operational member 81' to the movable member 82', a rotational shaft 88' is employed instead of the gear 83' (Figs. 27A and 27B).

The rotational shaft 88' includes ends 88A' and 88B' engaging the engagement portion 89A' of the operational member 81' and the engagement portion 89B' of the movable member 82', respectively.

In the example shown in Figs. 29A and 29B, the engagement portion 89A', 89B' of the operational member 81' and the movable member 82' include an elongated hole in which the end 88A', 88B' of the rotational shaft 88' can move up and down. In the example shown in Fig. 30, the engagement portion 89A', 89B' of the operational member 81' and the movable member 82' opens upward or downward so that the end 88A', 88B' of the rotational shaft 88' can move up and down.

In the disposal mechanisms 80B' and 80C', when a load is not applied to the operational member 81', the operational member 81' and the movable member 82' are located at the wait position, as indicated by solid lines in Figs. 29A and 30. When a load in the direction of D2 is applied to the operational member 81', the operational member 81' moves in the direction of D2, as indicated by double-dashed lines in Figs. 29B and 30. Since the operational member 81' and the movable member 82' are linked together via the rotational shaft 88', when the operational member 81' moves in the direction of D2, the movable member 82' moves in the opposite direction D2 from the operational member 81'. By this movement, the end 82B' of the movable member 82' pushes the end 90' of the analytical instrument 9' in the direction of D1, so that the analytical instrument 9' moves in the direction of D1. As a result, the analytical instrument 9' becomes removable from the analyzer 8'.

In the example shown in Figs. 29A and 29B, when the load applied to the operational member 81' in the direction of D2 is removed, the operational member 81' moves in the direction of D1 due to the elastic force of the spring 85' to return to the wait position. In accordance with this movement of the operational member 81', the movable member 82' moves in the direction of 2 to return to the wait position. In the example shown in Fig. 30, when the load applied to the operational member 81' in the direction of D2 is removed, the operational member 81' moves in the direction of D1 due to the elastic force of the spring 85' to return to the wait position, while the movable member 82' moves in the direction of D2 due to the elastic force of the spring 89' to return to the wait position.

In the example shown in Fig. 30, the spring 89' may be eliminated. In such a case, the movable member 82' does not move when the load applied to the operational member 81' is removed. However, when the analytical instrument 9' is inserted into the analyzer 3', the movable member 82' is pushed by the analytical instrument 9' and moved in the direction of D2 to be located at the wait position.

## Claims

1. An analyzer for analyzing a sample by using an analytical instrument taken out of a cartridge accommodating a plurality of analytical instruments, the analyzer comprising:
an operational member which is reciprocally movable relative to a housing; and
a movable member which reciprocates in accordance with reciprocal movement of the operational member, the reciprocating of the movable member being between a wait position and a take-out position for taking an analytical instrument out of the cartridge;
wherein the movable member comes into engagement with an analytical instrument accommodated in the cartridge for taking the analytical instrument out of the cartridge, with at least part of the analytical instrument received in the housing.

2. The analyzer according to claim 1, wherein the operational member moves due to a load applied thereto by the cartridge when the cartridge is mounted to the analyzer while being positioned relative to the analyzer.

3. The analyzer according to claim 2, wherein the movable member includes at least one arm for engagement with the analytical instrument.

4. The analyzer according to claim 3, wherein, when the cartridge is mounted to the analyzer, said at least one arm is inserted into the cartridge to engage an analytical instrument accommodated in the cartridge, and when the cartridge is detached from the analyzer, said at least one arm pulls and takes the analytical instrument out of the cartridge and guides at least part of the analytical instrument into the housing.

5. The analyzer according to claim 1, further comprising a detector for detecting whether or not the movable member is located at an appropriate position.

6. The analyzer according to claim 5, wherein the detector includes at least one switch, and
wherein the movable member includes at least one detection target portion for causing said at least one switch to generate an ON signal.

7. The analyzer according to claim 1, wherein each of the operational member and the movable member includes a rack portion including a plurality of cogs, and the operational member and the movable member are connected to each other via a gear, and
wherein, when the operational member moves, the movable member moves in an opposite direction from the operational member.

8. The analyzer according to claim 1, further comprising a stopper which restricts the movement of the movable member toward the wait position and positions the movable member at an analysis reference position set between the take-out position and the wait position when the analytical instrument taken out from the cartridge is held in engagement with the movable member.

9. The analyzer according to claim 1, wherein the movable member includes a pivotable portion which pivots in a direction crossing the movement direction of the movable member to apply a force in the crossing direction to the analytical instrument taken out of the cartridge when the movable member moves reciprocally.

10. The analyzer according to claim 9, further comprising a fixation element fixed to the housing,
wherein one of the pivotable portion and the fixation element includes a guide groove, whereas the other one of the pivotable portion and the fixation element includes a projection for engagement with the guide groove, and
wherein, when the movable member moves reciprocally relative to the housing, the projection moves within the guide groove so that the pivotable portion pivots.

11. The analyzer according to claim 1, further comprising an information recognizer for recognizing information as to the analytical instrument.

12. The analyzer according to claim 11, wherein the information recognizer includes a movable portion which is movable when the cartridge is mounted to the analyzer and a switch to be turned on or off by the movable portion.

13. A cartridge for accommodating a plurality of analytical instruments to be used for sample analysis at an analyzer, the cartridge being configured so that the analytical instruments are taken out by the analyzer when the cartridge is mounted to the analyzer, the cartridge comprising:
a main body for accommodating the analytical instruments;
a discharge port used for taking out the analytical instruments; and
a selector for enabling selection between a state in which the discharge port is exposed and a state in which the discharge port is not exposed.

14. The cartridge according to claim 13, wherein the selector is rotated or slid to enable selection between the state in which the discharge port is exposed and the state in which the discharge port is not exposed.

15. The cartridge according to claim 14, wherein the selector includes a rotational member which is capable of performing relative rotation.

16. The cartridge according to claim 14, wherein the selector includes an informative portion to which information as to the analytical instrument is applied.

17. The cartridge according to claim 16, wherein the informative portion is capable of outputting information to the analyzer when the cartridge is mounted to the analyzer with the discharge port exposed.

18. The cartridge according to claim 16, wherein intended information is applied to the informative portion by selecting whether or not a recess or a projection is to be formed at each of a plurality of predetermined regions of the selector.

19. The cartridge according to claim 16, wherein the information is related to sensitivity of the analytical instrument.

20. The cartridge according to claim 13, wherein the analyzer includes a movable member which is reciprocally movable to take an analytical instrument out of the cartridge and capable of being inserted through the discharge port, and
wherein each of the analytical instruments includes an engagement portion for engagement with the movable member.

21. The cartridge according to claim 20, wherein the engagement portion comprises a cutout.

22. The cartridge according to claim 13, further comprising a guide for controlling positional relationship between the cartridge and the analyzer in mounting the cartridge to the analyzer and enabling the cartridge to be mounted to an appropriate portion of the analyzer.

23. An analytical kit comprising: an analyzer for analyzing a sample by using an analytical instrument; and a cartridge accommodating a plurality of analytical instruments to be fed to the analyzer;
the analyzer comprising an operational member which is reciprocally movable relative to a housing, and
a movable member which reciprocates, in accordance with reciprocal movement of the operational member, between a wait position and a take-out position for taking an analytical instrument out of the cartridge,
wherein the movable member comes into engagement with an analytical instrument accommodated in the cartridge for taking the analytical instrument out of the cartridge, with at least part of the analytical instrument received in the housing.

24. The analytical kit according to claim 23, wherein the analytical instrument includes an engagement portion for engagement with the movable member.

25. The analytical kit according to claim 24, wherein the engagement portion comprises a cutout, and
wherein the movable member includes at least one arm for engagement with the cutout.

26. The analytical kit according to claim23, wherein the analyzer and the cartridge include mounting means for mounting the cartridge to an appropriate position of the analyzer.

27. The analytical kit according to claim26, wherein the mounting means comprises a projection provided at one of the analyzer and the cartridge and a recess provided at the other one of the analyzer and the cartridge.

28. The analytical kit according to claim 26, wherein, when the cartridge is mounted to the analyzer by utilizing the mounting means, the movable member moves due to a load applied to the operational member by the cartridge.

29. The analytical kit according to claim 23, wherein the cartridge further comprises an informative portion to which information as to the analytical instrument is applied, and
wherein the analyzer further comprises an information recognizer for recognizing information applied to the informative portion.

30. The analytical kit according to claim 29, wherein the cartridge includes a main body for accommodating the analytical instruments, a discharge port used for taking out the analytical instruments, and a selector for enabling selection between a state in which the discharge port is exposed and a state in which the discharge port is not exposed, and
wherein the informative portion is provided at the selector.

31. The analytical kit according to claim 30, wherein the selector includes a rotational member for enabling the selection between the state in which the discharge port is exposed and the state in which the discharge port is not exposed by rotating, and
wherein the informative portion is provided at the rotational member and outputs the information to the information recognizer when the cartridge is mounted to the analyzer with the discharge port exposed.

32. The analytical kit according to claim 31, wherein the information recognizer includes a plurality of movable portions which are movable when the cartridge is mounted to the analyzer, and switches to be turned on or off by the movable portions, and
wherein intended information is applied to the informative portion by selecting whether or not a recess or a projection is to be formed at each of a plurality of regions which are predetermined in the rotational member at positions corresponding to the movable portions.

33. The analytical kit according to claim 32, wherein information as to sensitivity of the analytical instrument is applied to the informative portion, and
wherein the information recognizer performs output for enabling selection of a corresponding calibration curve from a plurality of predetermined calibration curves based on the information.

34. An analyzer for use with an analytical instrument mounted thereto,
wherein the analyzer comprises a disposal mechanism for disposing of the analytical instrument mounted to the analyzer, the disposal mechanism comprising:
an operational member which is reciprocally movable relative to a housing; and
a movable member for disposing of the analytical instrument by moving at least partially in accordance with the reciprocal movement of the operational member.

35. The analyzer according to claim 34, wherein the movable member reciprocates, in accordance with reciprocal movement of the operational member, between a wait position and a disposal position at which the analytical instrument is to be disposed of.

36. The analyzer according to claim 35, wherein the disposal mechanism is configured so that when the operational member is moved in a load inputting direction from the disposal position toward the wait position, the movable member moves in a disposal direction from the wait position toward the disposal position which is opposite to the load inputting direction.

37. The analyzer according to claim 36, further comprising a link member for transmitting a load inputted to the operational member to the movable member.

38. The analyzer according to claim 37, wherein each of the operational member and the movable member includes a rack portion including a plurality of cogs, and
wherein the link member comprises a gear meshing with the cogs.

39. The analyzer according to claim 37, wherein the link member includes a first engagement portion engaging the operational member and a second engagement portion engaging the movable member and is rotatable about an intermediate portion between the first and the second engagement portions.

40. The analyzer according to claim 34, wherein the movable member is a rotational cam capable of engaging the analytical instrument and the operational member, and
wherein, due to the movement of the operational member, the rotational cam rotates and changes, by the rotation, an engagement position with the analytical instrument to move the analytical instrument.

41. The analyzer according to claim 34, wherein the operational member is biased in the disposal direction when moved in the load inputting direction.
